# EUROPÄISCHE PATENTSCHRIFT

(11) **EP 1 141 895 B1**
(45) Veröffentlichungstag und Bekanntmachung des Hinweises auf die Patenterteilung: **07.08.2002**
(21) Anmeldenummer: 00907417.0
(22) Anmeldetag: 05.01.2000
(51) Int. Cl.: G06T 7/60

(54) **VERFAHREN UND VORRICHTUNG ZUR BESTIMMUNG VON VOLUMINA IM MENSCHLICHEN UND TIERISCHEN KÖRPER**
METHOD AND DEVICE FOR DETERMINING VOLUMINA IN THE HUMAN OR ANIMAL BODY
PROCEDE ET DISPOSITIF POUR DETERMINER DES VOLUMES DANS LE CORPS HUMAIN OU ANIMAL

(30) Priorität: 08.01.1999 DE 19900414
(43) Veröffentlichungstag der Anmeldung: 10.10.2001
(73) Patentinhaber: Deutsches Krebsforschungszentrum Stiftung des öffentlichen Rechts, 69120 Heidelberg (DE)
(72) Erfinder: MAHR, Andreas, D-69214 Eppelheim (DE); BAHNER, Malte, D-69117 Dossenheim (DE); LEVEGRÜN, Sabine, New York, NY 10021 (US)
(74) Vertreter: Castell, Klaus, Dr.-Ing.
(86) Internationale Anmeldenummer: DE0000038
(87) Internationale Veröffentlichungsnummer: WO0041135

(56) Entgegenhaltungen:
- US-A- 4 856 528
- US-A- 4 961 425
- LONG D T ET AL: "COMPARATIVE EVALUATION OF IMAGE SEGMENTATION METHODS FOR VOLUME QUANTITATION IN SPECT" MEDICAL PHYSICS,US,AMERICAN INSTITUTE OF PHYSICS. NEW YORK, Bd. 19, Nr. 2, 1. März 1992 (1992-03-01), Seiten 483-489, XP000291143 ISSN: 0094-2405

## Beschreibung

Die Erfindung betrifft ein Verfahren und eine Vorrichtung zur Bestimmung von Volumina im menschlichen oder tierischen Körper. Dabei werden mittels eines geeigneten bildgebenden Verfahrens Bilddaten eines interessierenden Volumens erfasst und einer manuellen, semiautomatischen oder vollautomatischen Segmentierung unterzogen. Aus den segmentierten Bilddaten wird schließlich automatisch eine Größenangabe des interessierenden Volumens ermittelt.

Solche Verfahren und Vorrichtungen sind in unterschiedlichster Form bekannt. Sie liefern Informationen, die unter anderem von Arzten z.B. bei der Diagnose von Tumoren, bei der Therapiewahl und Therapieverlaufskontrolle sowie bei Organtransplantationen verwendet werden.

Die bekannten Verfahren und Vorrichtungen haben sich in der Praxis durchaus bewährt, und gerade bei der semiautomatischen und vollautomatischen Segmentierung wurden in den letzten Jahren große Fortschritte gemacht. Hierbei wird unter dem Begriff "Segmentierung" der Prozess verstanden, den man als "Bildverstehen" bezeichnet, nämlich der Übergang von einer Bildpunktmenge aus einer Vielzahl von Bildelementen (Pixel bzw. - hei einem Volumendatensatz - Voxel genannt) zu einer symbolischen Beschreibung (Tumor, Knochen etc.). Eine solche Segmentierung setzt sich bei diesem Verständnis aus einer Klassifizierung, nämlich der Bildung von Äquivalenzklassen im Merkmalsraum, und einer Identifizierung, also der Rücktransformation der Elemente einer Äquivalenzklasse aus dem Merkmalsraum in den Ortsraum, zusammen.

Die Druckschrift von David T. Long et al.: 'Comparatice evaluation of image segmentation methods for volume quantitation in SPECT' Medical Physics, US, American Institute of Physics, New York, Bd. 19, Nr. 2, 1. März 1992, Seiten 483-489 beschreibt und vergleicht verschiedene Bildsegmentierungsverfahren zur Volumenbestimmung im menschlichen Körper aus SPECT-Bildern (single photon emission CT-Bildern). Neben manuellen Verfahren werden auch vollautomatische Segmentierungsverfahren wie z.B. durch 3D-Kantendetektion beschrieben.

Der Erfindung liegt die Aufgabe zugrunde, ein Verfahren und eine Vorrichtung zur Bestimmung von Volumina im menschlichen oder tierischen Körper anzugeben, bei denen die Aussagekraft der ermittelten Werte und deren Interpretierbarkeit gegenüber den bekannten Verfahren und Vorrichtungen deutlich verbessert ist. Zudem sollen das neue Verfahren und die neue Vorrichtung problemlos in Kombination mit bzw. als Nachrüstung bei den bekannten Verfahren und Vorrichtungen eingesetzt werden können.

Die Aufgabe wird zum einen gelöst von einem Verfahren der eingangs genannten Art, bei welchem den Verfahrensschritten Erfassen von Bilddaten und Segmentieren der Bilddaten wenigstens ein Kennwert zugeordnet wird, der ein Maß für den Fehler dieser Verfahrensschritte ist, worauf aus dem zugeordneten Kennwert ein Fehlerwert als Maß für den Fehler bei der Ermittlung der Größenangabe bestimmt wird, wobei der Fehlerwert anzeig- bzw. ausgebbar ist. Vorzugsweise ist der Fehlerwert der Größenangabe zuordbar, so dass diese beiden Werte korreliert archiviert werden können.

Es ist auch möglich, den Verfahrensschritten Erfassen von Bilddaten und Segmentieren der Bilddaten jeweils wenigstens einen Kennwert zuzuordnen, der jeweils ein Maß für den Fehler des jeweiligen Verfahrensschrittes ist. Diese Kennwerte können dann zu einem Kennwert verknüpft und weiterverwendet oder aber getrennt weiterverwendet werden.

Die bekannten Verfahren und Vorrichtungen erwecken beim Benutzer oft den falschen Eindruck, die gelieferten Werte seien absolut genau, da Fehlergrenzen nicht angegeben werden. Dies kann fatale Folgen haben, zumal die Benutzer solcher Verfahren in der Regel stark eingespannte Ärzte sind, die schon von ihrer Ausbildung her nicht beurteilen können, an welcher Stelle des Ermittlungsprozesses welche Ungenauigkeiten insbesondere aufgrund physikalischer Gegebenheiten in den Endwert einfließen. Zudem ist aus Kostengründen eine schnelle Entscheidung bei der Interpretation der von den bekannten Verfahren und Vorrichtungen gelieferten Daten notwendig. So kann es vorkommen, dass bei der Therapieverlaufskontrolle das Volumen eines Tumors von einem bekannten Verfahren zunächst zu 50 ml, dann 48 ml, dann 43 ml ermittelt wird, was offensichtlich auf das Anschlagen der Therapie hindeutet. Tatsächlich kann aber aufgrund technischer, physikalischer und biologischer Besonderheiten die Unsicherheit der Messung unterschiedlich groß sein und z.B. bei der ersten Messung ± 2 ml, bei der zweiten ± 4 ml und bei der dritten ± 7 ml betragen, so dass also das tatsächliche Volumen des Tumors bei der ersten Messung nur 48 ml, bei der letzten aber 49 ml betragen haben kann, was dafür spricht, dass die gewählte Therapie, z.B. Chemotherapie, eben nicht anschlägt und dass stattdessen alsbald zu einer andere Therapieform, z.B. die Strahlentherapie, übergegangen werden sollte.

Das erfindungsgemäße Verfahren erhöht die Aussagekraft der so ermittelten Daten beträchtlich. Wird das Verfahren z.B. in der Medizin, insbesondere der Tumormedizin eingesetzt, so erlaubt die nunmehr mögliche Angabe von Fehlergrenzen dem behandelnden Arzt auch ohne detaillierte Kenntnisse der eingesetzten, in der Regel sehr aufwendigen Technik und deren Physik, die für die Therapieentscheidung und Therapieverlaufskontrolle wichtigen Volumeninformationen differenzierter beurteilen zu können, was eine wesentliche Erleichterung für den Entscheidungsprozeß bedeutet.

Das Verfahren hat den weiteren Vorteil, dass es bei allen Arten bildgebender Verfahren, z.B. CT, MRT, PET, Ultraschall etc., eingesetzt werden kann, wobei lediglich der oder die vom jeweiligen Verfahren abhängigen Kennwerte angepasst werden müssen. Je nach Art des verwendeten bildgebenden Verfahrens wird der wenigstens eine dem Verfahrensschritt des Erfassens von Bilddaten zugeordnete Kennwert üblicherweise wenigstens ein Maß aus der folgenden Gruppe von Maßen enthalten: Signal-Rausch-Verhältnis (z.B. je nach Gerät beeinflusst durch Röhrenspannung, Röhrenstrom, Rekonstruktionskernel), Gewebekontrast, Pitch und/oder Inkrement (bei der Spiral-CT), Sequenzparameter (bei der MRT), Schichtdicke, Matrixgröße und/oder eingesetzte Filter.

Erfolgt eine semiautomatische oder automatische Segmentierung, so kann der wenigstens eine dem Verfahrensschritt des Segmentierens zuzuordnende Kennwert ein Maß für die Genauigkeit eines beim Segmentieren verwendeten Segmentierungsverfahrens und/oder ein Maß für die Reproduzierbarkeit der Ergebnisse des verwendeten Segmentierungsverfahrens enthalten.

Erfolgt dagegen das Segmentieren manuell oder semiautomatisch, so wird zweckmäßigerweise jeder Person, die das Verfahren durchführt, ein persönlicher Kennwert zugeordnet und bei der Bestimmung des Fehlerwertes der Größenangabe berücksichtigt. Dies erlaubt es vorteilhaft, die unterschiedlichen Segmentierungsfähigkeiten der das Verfahren durchführenden Personen in die Ermittlung des Fehlerwertes einzubeziehen und damit zu berücksichtigen, dass die Aussagekraft der aus von erfahrenen Personen segmentierten Bilddatensätze ermittelten Volumina höher ist als diejenige von Volumina, die aus Bilddatensätze ermittelt wurden, die von noch unerfahrenen Personen segmentiert wurden.

Dabei kann der jeder Person zugeordnete persönliche Kennwert automatisch ermittelt werden, z.B. dadurch, dass die jeweilige Person bei vorgegebenen Testdaten eine oder mehrere manuelle oder semiautomatische Segmentierung/en auszuführen hat. Auf diese Weise kann ein selbstlernendes System realisiert werden, wobei vorteilhaft vorgesehen werden kann, dass die Bedienpersonen in regelmäßigen oder unregelmäßigen Intervallen Segmentierungen mit Testdaten ausführen. Durch Segmentierungen jeweils gleicher Testdaten lassen sich Schwankungen der einzelnen Bedienperson, die von einer Tagesform der Bedienperson oder ähnlichem abhängen, erfassen. Um Lerneffekte zu erfassen, können jeweils andere Testdaten zu Kennwertermittlung des persönlichen Kennwertes Verwendung finden. Es versteht sich, dass auch beide der vorgenannten Verfahren kumulativ genutzt werden können, das heißt, dass ein Teil der Testdaten mehrfach wiederholt segmentiert werden muss, während ein anderer Teil wesentlich seltener der Bedienperson vorgelegt wird. Ersterer Teil ermöglicht die Bestimmung beispielsweise einer Fehlerbandbreite, während letzterer Teil der Testdaten beispielsweise über Lerneffekte Auskunft geben kann.

Bei einer vorteilhaften Durchführungsform des Verfahrens wird dem interessierenden Volumen selbst wenigstens ein Kennwert zugeordnet und bei der Bestimmung des Fehlerwertes der Größenangabe berücksichtigt. Je nach Art des in dem interessierenden Volumen enthaltenen Gebildes, bei dem es sich z.B. um einen Tumor oder ein Organ handeln kann, spielen nämlich z.B. dessen Größe und/oder Gestalt eine unterschiedliche Rolle bei der Ermittlung der Genauigkeit bzw. des Fehlerintervalls des Volumenwertes.

Die genannte Aufgabe wird ferner gelöst von einer Vorrichtung zur Bestimmung von Volumina im menschlichen oder tierischen Körper mit Mitteln zum Einlesen von Bilddaten eines interessierenden Volumens, Mitteln zum manuellen, semiautomatischen oder vollautomatischen Segmentieren der Bilddaten und Mitteln zur automatischen Ermittlung einer Größenangabe des interessierenden Volumens aus den segmentierten Bilddaten, welche über wenigstens ein Datenspeicher verfügt, in dem Kennwerte abgelegt sind, die nach vorbestimmten Kriterien den eingelesenen und/oder den segmentierten Bilddaten zuordbar sind, wobei die Mittel zur automatischen Ermittlung der Größenangabe mit dem wenigstens einen Datenspeicher derart gekoppelt und derart ausgebildet sind, dass sie die Kennwerte aus dem Datenspeicher auslesen und aus den Kennwerten einen Fehlerwert als Maß für den Fehler bei der Ermittlung der Größenangabe bestimmen können.

Die Vorrichtung ist auch bei vorhandenen Systemen, insbesondere auch bei aufwendigen bildgebenden medizinischen Geräten wie z.B. einem Kernspinresonanzoder Computertomographen ohne größeren Aufwand nachrüstbar. Die Erfindung verbessert damit nicht nur die Aussagekraft, der von existierenden Geräten gelieferten Daten, sondern ist auch universell und kostengünstig einsetzbar.

Bei einer bevorzugten Ausführungsform der Erfindung sind in dem Datenspeicher auch ein den interessierenden Volumina selbst zugeordneter Kennwert abgelegt, so dass die Genauigkeit weiter erhöht und auch physikalische und biologische Besonderheiten berücksichtigt werden können.

Alternativ oder zusätzlich können in wenigstens einem mit den Mitteln zur Ermittlung der Größenangabe gekoppelten Datenspeicher Kennwerte für jede die Vorrichtung bedienende Person abgelegt sein, welche es dann erlauben, die individuellen Fähigkeiten der Personen beim Segmentieren bei der Ermittlung der Größenangabe und insbesondere eines der Größenangabe zugeordneten Fehlerintervalls zu berücksichtigen. Vorteilhaft kann dazu ein Datenspeicher mit Testdatensätzen vorgesehen sein, an denen die Vorrichtung bedienenden Personen eine manuelle oder semiautomatische Probesegmentierung vornehmen können, um so zu reproduzierbaren und vergleichbaren Aussagen über die individuellen Segmentaktionsfähigkeiten zu gelangen, wobei die Auswertung der Probesegmentierung und Ermittlung und Speicherung eines persönlichen Kennwertes für die jeweilige Person auch automatisch erfolgen kann.

Ist - was sich in vielen Einsatzfällen als zweckmäßig erwiesen hat - vorgesehen, dass die individuellen Segmentierungsfähigkeiten der Bedienpersonen in regel- oder unregelmäßigen Intervallen erneut getestet werden, so kann erfindungsgemäß den persönlichen Kennwerten ein Datensatz zugeordnet werden, der den oder die zur Ermittlung des jeweiligen Kennwertes verwendeten Testdatensatz/-sätze identifiziert.

Auf diese Weise kann vorteilhaft gewährleistet werden, dass bei erneuten Testsegmentierungen ein und dieselbe Person einen definierten Testdatensatz erhält. So kann der Testdatensatz beispielsweise Testdaten enthalten, die dieser Person wiederholt vorgelegt werden, um Aussagen über die Fehlerbandbreite dieser Person bei der Segmentierung treffen zu können. Weiterhin kann der Testdatensatz Testdaten enthalten, die nur einmalig oder in größeren Abständen der Person vorgelegt werden, so dass auch Trainingseffekte, die durch häufiges Segmentieren identischer Testdaten bedingt sind, bzw. ähnliche Effekte erfasst werden können. Es ist beispielsweise auch möglich, in einem Testdatensatz jeweils nur ein oder zwei bzw. sehr wenige Testdaten für jede erneute Testsegmentierung auszutauschen, während die übrige beibehalten werden.

Im Regelfall wird es zweckmäßig sein, die Vorrichtung mit Mitteln zum Darstellen und/oder Auslesen der ermittelten Größenangabe und des ermittelten Fehlerwertes zu versehen, wobei insbesondere an Monitore, Drucker, Festplatten, CD-ROMs und Disketten zu denken ist.

Im Rahmen des Erfindungsgedankens sind zahlreiche Abwandlungen und Weiterbildungen möglich, die sich z.B. auf die Art und Ermittlung der Kennwerte und des daraus ermittelten Maßes für den Fehler der automatisch ermittelten Größenangabe beziehen. Erfindungswesentlich ist jedenfalls, dass bei der Volumenbestimmung zusätzlich zur Größenangabe auch ein Fehlerwert für die Größenangabe ermittelt und spezifiziert wird.

Nachfolgend wird anhand einer semiautomatischen Segmentation, welche den komplexesten Fall der Umsetzung der vorliegenden Erfindung darstellt, eine mögliche Realisierung der Erfindung dargestellt. Hierbei ist zu unterscheiden zwischen benutzerunabhängigen und benutzerabhängigen Fehlern. Während bei einer manuellen Segmentation lediglich benutzerabhängige Fehler ausschlaggebende Deutung erlangen, sind bei einer vollautomatischen Segmentation lediglich benutzerunabhängige Fehler von Bedeutung. Bei einer semiautomatischen Segmentation hingegen spielen beide Fehlerarten eine Rolle.

Des weiteren ist zu unterscheiden zwischen unabhängigen und voneinander abhängigen Fehlern. Unabhängige Fehler können im Rahmen einzelner Kennwerte einer bestimmten Fehlerquelle zugeordnet werden. Diese einzelnen Kennwerte können beispielsweise als Fehlerangabe oder aber als Faktor mittels Fehlerverknüpfungsverfahren oder einfache multiplikative Verknüpfung in die Gesamtfehlerberechnung eingehen. Erfahrungsgemäß können jedoch auch voneinander abhängige Fehler, wie beispielsweise das Signal-Rausch-Verhältnis und der Gewebekontrast, vorliegen. Hierbei äußert sich diese Abhängigkeit dahingehend, dass bei einer semiautomatischen bzw. manuellen Segmentation Veränderungen dieser Faktoren eine Veränderung des Segmentationsfehlers bedingen, die nicht lediglich einem der Faktoren zugeordnet werden können. Der durch derartige voneinander abhängige Größen bedingte Fehler kann beispielsweise im Rahmen einer Tabelle in einem Datenspeicher abgelegt sein. Hierbei ist die Dimensionalität der Tabelle durch die Zahl der voneinander abhängigen Größen definiert, und die Tabelle enthält in jedem Tabellenfeld einen Kennwert bzw. Fehlerwert, der entsprechenden Spalten dieser Tabelle entspricht.

Diese Kennwerte bzw. Fehlerwerte werden vorab experimentell ermittelt. Dieses kann beispielsweise aufgrund von Probesegmentationen durch eine große Anzahl von Ärzten geschehen.

Um nunmehr den Fehler bei einer konkreten Segmentation zu bestimmen, werden zunächst die aktuellen Werte der voneinander abhängigen Größen, wie Signal-Rausch-Verhältnis und Gewebekontrast, ermittelt. Dieses kann beispielsweise durch eine Online-Berechnung oder durch eine Messung erfolgen. Im Anschluss hieran wird der zu diesen Größen zugehörige Fehler aus der Tabelle ausgelesen. Hierbei können zwischen einzelnen Tabellenfeldern je nach Erfordernissen Interpolationen vorgenommen werden.

Der auf dieser Weise bestimmte Kennwert bzw. Fehlerwert kann anschließend noch mit unabhängigen Kenn- bzw. Fehlerwerten verknüpft werden, um einen Gesamtfehler zu bestimmen. Diese unabhängigen Kenn- bzw. Fehlerwerte ihrerseits können ebenfalls aus einem Zusammenspiel mehrere Größen bestimmt sein. Als derartige unabhängige Kenn- bzw. Fehlerwerte kommen insbesondere ein algorithmusabhängiger Fehlerwert für den bei der semiautomatischen Segmentation angewandten Algorithmus sowie ein benutzerabhängiger Kenn- bzw. Fehlerwert in Frage.

Es ist darüber hinaus auch möglich, einige oder sämtliche der voneinander abhängigen Größen benutzerabhängig zu speichern. Hierfür kann zum Beispiel eine vorbeschriebene Tabelle für jeden der Benutzer abgespeichert werden. Je mehr Größen aus der benutzerunabhängigen Tabelle in die benutzerabhängige Tabelle verlagert werden können, desto genauer kann die Fehlerangabe erfolgen. Andererseits hat diese Vorgehensweise den Nachteil, dass der einzelne Nutzer bzw. Arzt eine verhältnismäßig große Zahl an Testdaten zu segmentieren hat.

Hierbei ist zu betonen, dass es sich bei dem erfindungsgemäßen Verfahren bzw. der erfindungsgemäßen Vorrichtung nicht um eine Messfehlerkorrektur handelt. Vielmehr ermöglicht das erfindungsgemäße Verfahren bzw. die erfindungsgemäße Vorrichtung erstmals, dass ein ohnehin in jeder Messapparatur vorhandener Fehler bei den gattungsgemäßen Verfahren bzw. Vorrichtungen in relevantem Maße in eine Begutachtung des Messergebnisses einfließt bzw. einfließen kann.

Weitere Vorteile, Ziele und Eigenschaften vorliegender Erfindung werden anhand nachfolgender Erläuterung anliegender Zeichnung dargestellt, in welcher beispielhaft der Verfahrensablauf bei einer Volumenberechnung verdeutlicht ist. In der Zeichnung zeigen:

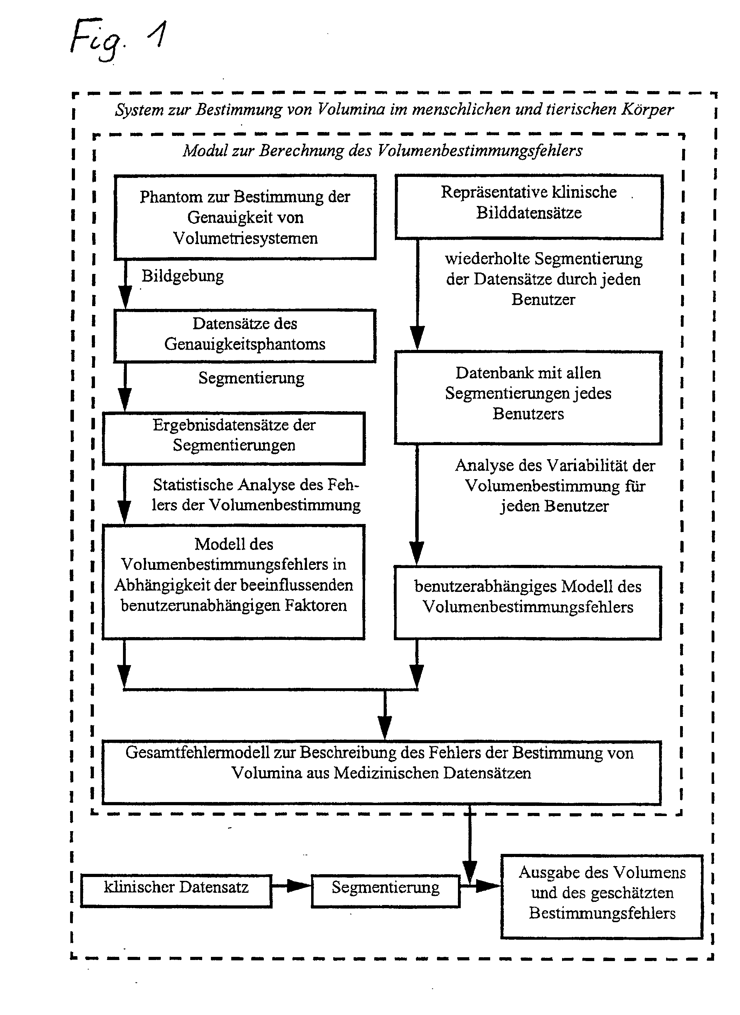

- Figur 1: ein Flussdiagramm für den Ablauf einer Volumenberechnung und

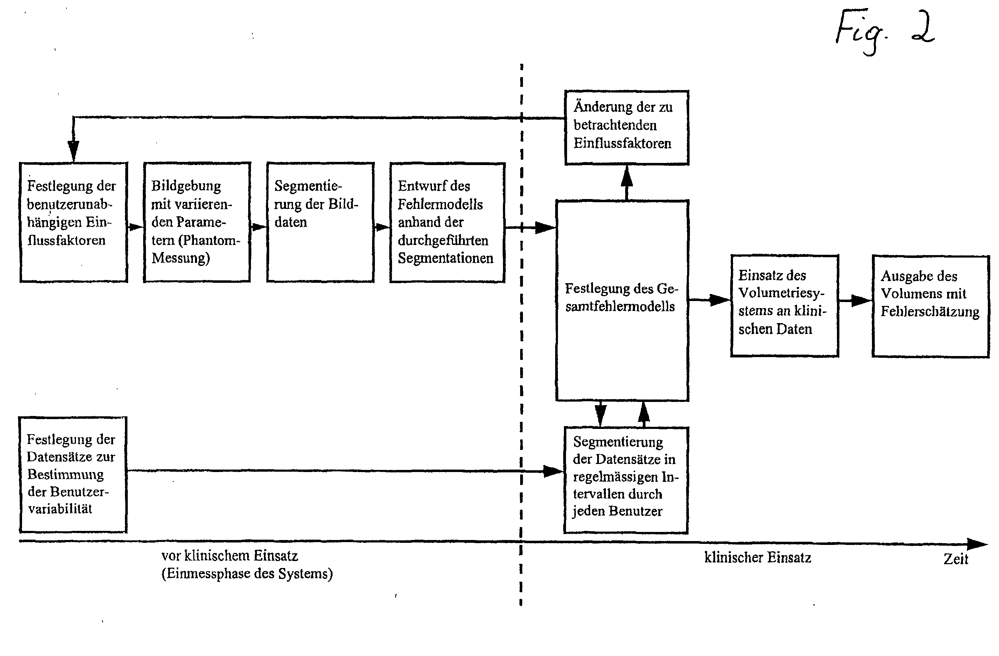

- Figur 2: deren zeitlicher Verlauf.

Das in den Figuren dargestellte Ausführungsbeispiel bezieht sich auf die Volumetrie von Tumoren, insbesondere von Lebertumoren bei einer Bildgebung mit einem Spiral-CT. Hierbei wird zur Volumetrie ein semiautomatischer Segmentationsalgorithmus genutzt. Hieraus folgt, dass sowohl ein benutzerabhängiger als auch ein benutzerunabhängiger Einfluss auf die Genauigkeit der Volumenbestimmung existiert.

Insofern müssen sowohl benutzerabhängige also auch benutzerunabhängige Fehlerkomponenten berücksichtigt werden, wie dieses aus den beiden Diagrammen deutlich ersichtlich ist. Als Einflussfaktoren konnten hierbei beispielsweise folgende Parameter identifiziert werden: als Objektparameter die Größe des Tumors; als Bildgebungsparameter der Kontrast des Tumors und als Segmentierungsparameter: der eingesetzte Segmen-tationsalgorithmus.

In einer ersten Nährung können alle übrigen Parameter als konstant betrachtet werden, wobei es sich versteht, dass ohne weiteres auch andere Parameter als beeinflussend angesehen und entsprechend behandelt werden können.

Um einen benutzerunabhängigen Fehler zu bestimmen, wird mit Hilfe eines CT-Phantoms mit unterschiedlich großen Testobjekten und unterschiedlichen Dichten eine Testreihe mit dem Spiral-CT unter den in der Praxis bei einer Abdomenbildgebung üblichen Einstellungen vorgenommen. Die auf diese Weise gewonnenen Daten werden mit Hilfe des verwandten Segmentationsalgorithmusses konturiert. Um hierbei die Benuizerunabhängigkeil zu garantieren, wird die Segmentierung entweder durch eine ausreichend große Anzahl an Personen oder in ausreichend großer Wiederholungsanzahl durch eine Person durchgeführt. Es versteht sich, dass der Begriff "ausreichend" durch eine statistische Konvergenz definiert ist. Durch mathematisches Fitten wird aus den so erhaltenen Volumendaten eine in vorliegendem Fall zweidimensionale Funktion f(x,y) gewonnen, welche den Fehler der Volumenbestimmung (f) in Abhängigkeit der Objektgröße (x) und des Kontrastes (y) beschreibt.

Um den benutzerabhängigen Fehler zu bestimmen, werden drei repräsentative Testdatensätze mit klinischen Fällen ausgewählt, welche in monatlichem Abstand durch jeden Benutzer segmentiert werden müssen.

Die Ergebnisses dieser Segmentierungen werden gespeichert. Bei der Nutzung des Volumetriesystems werden online die Variabilität aller Ergebnisse berechnet und die Standartabweichung als Maß für den benutzerabhängigen Fehler herangezogen. Es versteht sich in diesem Zusammenhang, dass auch andere statistische Vorgehensweisen diesbezüglich denkbar sind.

Die Verknüpfung des benutzerunabhängigen Fehlers und des benutzerabhängigen Fehlers - im Zweifelsfall durch entsprechende, bekannte mathematische Maßnahmen - führt auf diese Weise zu einer Gesamtfehlerfunktion, die für jeden Benutzer individuell angepasst ist.

Führt der Benutzer im Anschluss hieran eine Volumenbestimmung an einen klinischen Datensatz durch, wird eine Abschätzung des Fehlers dieser an dem klinischen Datensatz durchgeführten Bestimmung anhand der Parameter Kontrast, Größe des Tumors und Benutzereinfluss vorgenommen. Das Ergebnis wird bei vorliegendem Ausführungsbeispiel in der Form Volumen = xxx,xx ml +/- xx,xx ml ausgegeben. Auf diese Weise kann ein Therapieverlauf wesentlich genauer kontrolliert werden, da der Benutzer auch eine Aussage über die Genauigkeit der jeweiligen Messung erhält. Dieses ist insbesondere dann von Bedeutung , wenn beispielsweise das Tumorvolumen während einer Behandlung scheinbar sinkt, während der entsprechende Fehler überproporlional ansteigt, sodass im Rahmen der Messgenauigkeit sogar ein Anstieg des Tumorvolumens denkbar wäre. In einem derartigen Fall wären dann geeignete Maßnahmen, die entsprechend die Messgenauigkeit erhöhen, durchzuführen, bevor von einem Therapieerfolg gesprochen werden könnte.

## Patentansprüche

1. Verfahren zur Bestimmung von Volumina im menschlichen oder tierischen Körper, wobei mittels eines geeigneten bildgebenden Verfahrens Bilddaten eines interessierenden Volumens erfasst werden, die erfassten Bilddaten manuell, semiautomatisch oder vollautomatisch segmentiert werden und wobei aus den segmentierten Bilddaten automatisch eine Größenangabe des interessierenden Volumens ermittelt wird, ***dadurch gekennzeichnet,***
**dass** den Verfahrensschritten Erfassen von Bilddaten und Segmentieren der Bilddaten wenigstens ein zuvor bestimmter Kennwert zugeordnet wird, der ein Maß für den Fehler dieser Verfahrensschritte ist,
**dass** aus dem zugeordneten Kennwert ein Fehler als Maß für den Fehler bei der Ermittlung der Größenangabe bestimmt wird und
**dass** der Fehlerwert, vorzugsweise unter Zuordnung mit der Größenangabe, anzeig- bzw. ausgebbar ist.

2. Verfahren nach Anspruch 1, ***dadurch gekennzeichnet, dass*** auch dem interessierenden Volumen selbst wenigstens ein Kennwert zugeordnet und bei der Bestimmung des Fehlerwertes der Größenangabe berücksichtigt wird.

3. Verfahren nach Anspruch 1 oder 2, wobei das Segmentieren manuell oder semiautomatisch erfolgt, ***dadurch gekennzeichnet, dass*** jeder Person, die das Verfahren durchführt, wenigstens ein persönlicher Kennwert zugeordnet und bei der Bestimmung des Fehlerwertes der Größenangabe berücksichtigt wird.

4. Verfahren nach Anspruch 3, ***dadurch gekennzeichnet, dass*** der jeder Person zugeordnete persönliche Kennwert automatisch ermittelt wird.

5. Verfahren nach Anspruch 4, ***dadurch gekennzeichnet, dass*** die automatische Ermittlung des einer Person zuzuordnenden Kennwertes anhand einer von der Person bei vorgegebenen Testdaten durchgeführten manuellen oder semiautomatischen Segmentierung erfolgt.

6. Verfahren nach einem der Ansprüche 1 bis 5, ***dadurch gekennzeichnet, dass*** der wenigstens eine dem Verfahrensschritt des Erfassens von Bilddaten zugeordnete Kennwert wenigstens ein Maß aus der folgenden Gruppe von Maßen enthält: Signal-Rausch-Verhältnis, Gewebekontrast, Pitch, Inkrement, Sequenzparameter, Schichtdicke, Matrixgröße, eingesetzte Filter.

7. Verfahren nach einem der Ansprüche 1 bis 6, wobei eine semiautomatische oder automatische Segmentierung erfolgt, ***dadurch gekennzeichnet, dass*** der wenigstens eine dem Verführensschritt des Segmentierens zuzuordnende Kennwert ein Maß für die Genauigkeit eines beim Segmentieren verwendeten Segmentierungsverfahrens und/oder ein Maß für die Reproduzierbarkeit der Ergebnisse des verwendeten Segmentierungsverfahrens enthält.

8. Verfahren nach Anspruch 2, ***dadurch gekennzeichnet, dass*** der wenigstens eine dem interessierenden Volumen zuzuordnende Kennwert ein Maß für die Größe und/oder Gestalt des interessierenden Volumens enthält.

9. Verfahren nach einem der Ansprüche 1 bis 8, ***dadurch gekennzeichnet, dass*** das interessierende Volumen das Volumen eines Tumors ist.

10. Verfahren nach einem der Ansprüche 1 bis 8, ***dadurch gekennzeichnet, dass*** das interessierende Volumen das Volumen eines Organs ist.

11. Vorrichtung zur Bestimmung von Volumina im menschlichen oder tierischen Körper mit Mitteln zum Einlesen von Bilddaten eines interessierenden Volumens, mit Mitteln zum manuellen, semiautomatischen oder vollautomatischen Segmentieren der Bilddaten und mit Mitteln zur automatischen Ermittlung einer Größenangabe des interessierenden Volumens aus den segmentierten Bilddaten, ***dadurch gekennzeichnet, dass***
wenigstens ein Datenspeicher vorgesehen ist,
in dem wenigstens einen Datenspeicher Kennwerte abgelegt sind, die nach vorbestimmten Kriterien den eingelesenen und/oder den segmentierten Bilddaten zuordenbar sind, und
die Mittel zur automatischen Ermittlung der Größenangabe mit dem wenigstens einen Datenspeicher derart gekoppelt und derart ausgebildet sind,
sie die Kennwerte aus dem Datenspeicher auslesen und aus den Kennwerten einen Fehlerwert als Maß für den Fehler bei der Ermittlung der Größenangabe bestimmen können.

12. Vorrichtung nach Anspruch 11, ***dadurch gekennzeichnet, dass*** in dem Datenspeicher ein dem interessierenden Volumen selbst zugeordneter Kennwert abgelegt ist.

13. Vorrichtung nach Anspruch 11 oder 12, ***dadurch gekennzeichnet, dass*** Mittel zum Darstellen und/oder Auslesen der ermittelten Größenangabe und des ermittelten Fehlerwertes vorgesehen sind.

14. Vorrichtung nach einem der Ansprüche 11 bis 13, ***dadurch gekennzeichnet, dass*** in wenigstens einem mit den Mitteln zur Ermittlung der Größenangabe gekoppelten Datenspeicher Kennwerte für jede die Vorrichtung bedienende Person abgelegt sind.

15. Vorrichtung nach Anspruch 14, ***dadurch gekennzeichnet, dass*** ein Datenspeicher mit Testdatensätzen vorgesehen ist, an denen die Vorrichtung bedienenden Personen eine manuelle oder semiautomatische Probesegmentierung vornehmen können.

16. Vorrichtung nach Anspruch 15, ***dadurch gekennzeichnet, dass*** Mittel zur Auswertung der Probesegmentierung und Ermittlung und Speicherung eines persönlichen Kennwertes für die jeweilige Person vorgesehen sind.

17. Vorrichtung nach Anspruch 16, ***dadurch gekennzeichnet, dass*** den persönlichen Kennwerten ein Datensatz zugeordnet ist, der den oder die zur Ermittlung des jeweiligen Kennwertes verwendeten Testdatensatz/Testdatensätze identifiziert.

18. Bildgebendes medizinisches Gerät mit einer Vorrichtung nach einem der Ansprüche 11 bis 17.

19. Verwendung einer Vorrichtung oder eines medizinischen Gerätes nach einem der Ansprüche 11 bis 18 zur Ermittlung des Volumens eines Tumors.

20. Verwendung einer Vorrichtung oder eines medizinischen Gerätes nach einem der Ansprüche 11 bis 18 zur Ermittlung des Volumens eines Organs.

## Claims

1. A method for determining volumina in the human or animal body, whereby image data of an interesting volume are acquired by means of a suitable image-generating method, the acquired image data are segmented manually, semi-automatically or fully automatically and whereby dimensional information of the interesting volume is determined automatically from the segmented image data, **characterised in that**
the procedure steps of image data acquisition and image data segmentation are allocated at least one pre-determined characteristic value, which is a measure of the error of these procedure steps,
an error as a measure of the error in the determination of the dimensional information is determined from the allocated characteristic value and
the error value can be displayed or outputted, preferably in association with the dimensional information.

2. The method according to claim 1, **characterised in that** at least one characteristic value is also allocated to the interesting volume itself and is taken into account in the determination of the error value of the dimensional information.

3. The method according to claim 1 or 2, whereby the segmentation takes place manually or semi-automatically, **characterised in that** at least one personal characteristic value is allocated to each person who performs the method and is taken into account in the determination of the error value of the dimensional information.

4. The method according to claim 3, **characterised in that** the personal characteristic value allocated to each person is determined automatically.

5. The method according to claim 4, **characterised in that** the automatic determination of the characteristic value allocated to a person takes place on the basis of a manual or semi-automatic segmentation carried out by the person with preset test data.

6. The method according to any one of claims 1 to 5, **characterised in that** the at least one characteristic value allocated to the procedure step of image data acquisition contains at least one measure from the following group of measures: signal-noise ratio, tissue contrast, pitch, increment, sequence parameters, layer thickness, matrix size, filters used.

7. The method according to any one of claims 1 to 6, whereby a semi-automatic or automatic segmentation takes place, **characterised in that** the at least one characteristic value allocated to the procedure step of segmentation contains a measure of the accuracy of a segmentation procedure used in the segmentation and/or a measure of the reproducibility of the results of the segmentation procedure used.

8. The method according to claim 2, **characterised in that** the at least one characteristic value allocated to the interesting volume contains a measure of the size and/or shape of the interesting volume.

9. The method according to any one of claims 1 to 8, **characterised in that** the interesting volume is the volume of a tumour.

10. The method according to any one of claims 1 to 8, **characterised in that** the interesting volume is the volume of an organ.

11. A device for determining volumina in the human or animal body with means for the reading-in of image data of an interesting volume, with means for the manual, semi-automatic or fully automatic segmentation of the image data and with means for the automatic determination of dimensional information of the interesting volume from the segmented image data,
**characterised in that**
at least one data memory is provided,
characteristic values, which can be allocated according to predetermined criteria to the read-in and/or the segmented image data, are filed in the at least one data memory and
the means for the automatic determination of the dimensional information are coupled with the at least one data memory in such a way and designed in such a way
they can read out the characteristic values from the data memory and can determine from the characteristic values an error value as a measure of the error in the determination of the dimensional information.

12. The device according to claim 11, **characterised in that** a characteristic value allocated to the interesting volume itself is filed in the data memory.

13. The device according to claim 11 or 12, **characterised in that** means are provided for displaying and/or reading-out the determined dimensional information and
the determined error value.

14. The device according to any one of claims 11 to 13, **characterised in that** characteristic values for each person using the device are filed in at least one data memory coupled with the means for determining the dimensional information.

15. The device according to claim 14, **characterised in that** a data memory is provided with test data records, on which persons using the device can carry out a manual or semi-automatic trial segmentation.

16. The device according to claim 15, **characterised in that** means are provided for evaluating the trial segmentation and determining and storing a personal characteristic value for the respective person.

17. The device according to claim 16, **characterised in that** a data record, which identifies the test data record/ records used to determine the respective characteristic value, is allocated to the personal characteristic values.

18. An image-generating medical apparatus with a device according to any one of claims 11 to 17.

19. The use of a device or a medical apparatus according to any one of claims 11 to 18 for determining the volume of a tumour.

20. The use of a device or a medical apparatus according to any one of claims 11 to 18 for determining the volume of an organ.

## Revendications

1. Procédé pour déterminer des volumes dans le corps humain ou animal, des données graphiques d'un volume intéressant étant détectées au moyen d'un procédé adéquat d'élaboration d'image, les données graphiques détectées étant segmentées de manière manuelle, semi-automatique ou entièrement automatique et une indication de taille du volume intéressant étant calculée automatiquement à partir des données graphiques segmentées, **caractérisé en ce que**
aux étapes de détection des données graphiques et de segmentation des données graphiques est associée au moins une valeur caractéristique définie au préalable qui est une marge d'erreur pour cette étape,
à partir de la valeur caractéristique associée, une erreur est définie comme marge d'erreur lors du calcul de l'indication de taille et
que la marge d'erreur, à laquelle est de préférence associée l'indication de la taille, peut être affichée ou éditée.

2. Procédé selon la revendication 1, **caractérisé en ce qu**'au volume intéressant lui-même est associée également au moins une valeur caractéristique et que celle-ci est prise en compte lors de la détermination de la marge d'erreur de l'indication de taille.

3. Procédé selon la revendication 1 ou 2, la segmentation se faisant de manière manuelle ou automatique, **caractérisé en ce qu**'à chaque personne qui réalise le procédé est attribuée au moins une valeur caractéristique personnelle et que celle-ci est prise en compte lors de la détermination de la marge d'erreur de l'indication de taille.

4. Procédé selon la revendication 3, **caractérisé en ce que** la valeur caractéristique personnelle attribuée à chaque personne est calculée automatiquement.

5. Procédé selon la revendication 4, **caractérisé en ce que** le calcul automatique d'une valeur caractéristique à attribuer à une personne est effectué sur la base d'une segmentation réalisée de manière manuelle ou semi-automatique par la personne avec des données d'essai prédéfinies.

6. Procédé selon une des revendications 1 à 5,
**caractérisé en ce que** la valeur caractéristique au minimum à associer à l'étape de détection des données graphiques comporte au moins une mesure du groupe de mesures suivantes : rapport signal-bruit, contraste de tissu, pas, incrémentation, paramètre de séquence, épaisseur de couche, taille de matrice, filtre utilisé.

7. Procédé selon une des revendications 1 à 6, une segmentation semi-automatique ou automatique ayant lieu, **caractérisé en ce que** la valeur caractéristique au minimum à associer à l'étape de segmentation comporte une mesure pour l'exactitude d'un procédé de segmentation utilisé lors de la segmentation et/ou une mesure pour la reproductibilité des résultats du procédé de segmentation utilisé.

8. Procédé selon la revendication 2, **caractérisé en ce que** la valeur caractéristique au minimum à associer au volume intéressant comporte une mesure pour la taille et/ou la forme du volume intéressant.

9. Procédé selon une des revendications 1 à 8, **caractérisé en ce que** le volume intéressant est le volume d'une tumeur.

10. Procédé selon une des revendications 1 à 8, **caractérisé en ce que** le volume intéressant est le volume d'un organe.

11. Dispositif pour déterminer des volumes dans le corps humain ou animal avec des moyens de lecture de données graphiques d'un volume intéressant, avec des moyens de segmentation manuelle, semi-automatique ou entièrement automatique des données graphiques et avec des moyens de calcul automatique d'une indication de taille du volume intéressant à partir des données graphiques segmentées, **caractérisé en ce que**
au moins une mémoire est prévue,
dans laquelle des valeurs caractéristiques qui peuvent être associées selon des critères prédéfinis aux données graphiques lues et/ou segmentées sont stockées, et
les moyens de calcul automatique de l'indication de taille sont couplés avec cette mémoire prévue au minimum et conçus de manière à ce
qu'ils lisent les valeurs caractéristiques dans la mémoire et puissent déterminer à partir des valeurs caractéristiques une valeur d'erreur comme marge d'erreur lors du calcul de l'indication de taille.

12. Dispositif selon la revendication 11, **caractérisé en ce que**, dans la mémoire, une valeur caractéristique associée au volume intéressant lui-même est stockée.

13. Dispositif selon la revendication 11 ou 12, **caractérisé en ce que** des moyens de représentation et/ou de lecture de l'indication de taille calculée et de la marge d'erreur calculée sont prévus.

14. Dispositif selon une des revendications 11 à 13, **caractérisé en ce que,** dans au moins une mémoire couplée aux moyens de calcul de l'indication de taille, des valeurs caractéristiques associées pour chaque personne utilisant le dispositif sont stockées.

15. Dispositif selon la revendication 14, **caractérisé en ce qu**'est prévue une mémoire avec des articles d'essai, avec lesquels les personnes utilisant le dispositif peuvent réaliser un essai de segmentation manuelle ou semi-automatique.

16. Dispositif selon la revendication 15, **caractérisé en ce que** des moyens d'exploitation de l'essai de segmentation et de calcul et de mémorisation d'une valeur caractéristique personnelle sont prévus pour la personne respective.

17. Dispositif selon la revendication 16, **caractérisé en ce qu**'est associé aux valeurs caractéristiques un article qui identifie l'article/les articles d'essai utilisés pour le calcul de la valeur caractéristique respective.

18. Appareil médical fournissant une image avec un dispositif selon une des revendications 11 à 17.

19. Utilisation d'un dispositif ou d'un appareil médical selon une des revendications 11 à 18 pour calculer le volume d'une tumeur.

20. Utilisation d'un dispositif ou d'un appareil médical selon une des revendications 11 à 18 pour calculer le volume d'un organe.
